# EUROPEAN PATENT APPLICATION

(11) **EP 4 737 432 A1**
(43) Date of publication of application: **06.05.2026**
(21) Application number: 25845564.1
(22) Date of filing: 30.07.2025
(51) Int. Cl.: C07C 17/354, B01J 23/44, C07B 61/00, C07C 19/08

(54) **METHOD FOR PRODUCING FLUOROETHANE**

(30) Priority: 31.07.2024 JP 2024124566
(71) Applicant: Daikin Industries, Ltd., Osaka-shi, Osaka 530-0001 (JP)
(72) Inventor: TAKAHASHI, Kazuhiro, Osaka-Shi, Osaka 530-0001 (JP); SHIMADA, Tomoo, Osaka-Shi, Osaka 530-0001 (JP)
(74) Representative: Hoffmann Eitle
(86) International application number: PCT/JP2025/027090
(87) International publication number: WO 2026/029123

(57) **Abstract**

A method for producing a fluoroethane represented by the following formula (1):

CH₂X¹CHX²X³ (1)

wherein X¹, X², and X³ each independently represent a hydrogen atom or a fluorine atom, and at least one of X¹, X², and X³ represents a fluorine atom,
the method comprising step A of obtaining the fluoroethane from a chlorofluoroethane represented by the following formula (2) in the presence of a catalyst:

CX⁴ClFCX⁵X⁶X⁷ (2)

wherein X⁴, X⁵, X⁶, and X⁷ each independently represent a hydrogen atom, a fluorine atom, or a chlorine atom, and at least one of X⁵, X⁶, and X⁷ represents a hydrogen atom.

## Description

### Technical Field

The present disclosure relates to a method for producing a fluoroethane.

### Background Art

Fluoroethanes typified by 1,1,2-trifluoroethane (which hereinafter may be simply referred to as "HFC-143" in the present specification) are known as a starting material for producing various refrigerants. Various methods have been proposed for the production of fluoroethanes such as HFC-143.

For example, Patent Literature 1 (PTL 1) proposes a technique for producing chlorotrifluoroethylene or trifluoroethylene by a hydrogenation reaction of 1,1,2-trichloro-1,2,2-trifluoroethylene (which hereinafter may be simply referred to as "CFC-113" in the present specification) or the like, in the presence of a hydrogenation catalyst.

However, when a fluoroethane, such as HFC-143, is produced by the method disclosed in PTL 1, there is a problem in that the selectivity of the desired product is low. Furthermore, there is no disclosure of a method for obtaining a hydrofluoroethane from a hydrochlorofluorocarbon (which hereinafter may be simply referred to as "HCFC" in the present specification).

### Citation List

### Patent Literature

PTL 1: JPH4-117333A

### Summary of Invention

### Technical Problem

In view of the circumstances described above, an object of the present disclosure is to provide a method for obtaining a hydrofluoroethane with high selectivity using a hydrochlorofluoroethane as a starting material.

### Solution to Problem

The present inventors conducted extensive research to achieve the above object, and found that a fluoroethane can be obtained in a high yield by using a catalyst. Based on this finding, the inventors continued further research and have accomplished the present disclosure.

Specifically, the present disclosure provides the following methods for producing a fluoroethane.

### Item 1.

A method for producing a fluoroethane represented by the following formula (1):

CH₂X¹CHX²X³ (1)

wherein X¹, X², and X³ each independently represent a hydrogen atom or a fluorine atom, and at least one of X¹, X², and X³ represents a fluorine atom,
the method comprising step A of obtaining the fluoroethane from a chlorofluoroethane represented by the following formula (2) in the presence of a catalyst:

CX⁴ClFCX⁵X⁶X⁷ (2)

wherein X⁴, X⁵, X⁶, and X⁷ each independently represent a hydrogen atom, a fluorine atom, or a chlorine atom, and at least one of X⁵, X⁶, and X⁷ represents a hydrogen atom.

### Item 2.

The method according to Item 1, wherein the catalyst is at least one member selected from the group consisting of Ni, Pd, Pt, Rh, and Ru.

### Item 3.

The method according to Item 1 or 2, wherein the catalyst is supported on a carrier, and the carrier is a carbon-based carrier.

### Item 4.

The method according to any one of Items 1 to 3, wherein the fluoroethane is 1,1,2-trifluoroethane (HFC-143), 1,1-difluoroethane (HFC-152a), 1,2-difluoroethane (HFC-152), or fluoroethane (HFC-161).

### Item 5.

The method according to any one of Items 1 to 4, wherein the chlorofluoroethane is 1,2-dichloro-1,1,2-trifluoroethane (HCFC-123a) or 1,1-dichloro-1,2,2-trifluoroethane (HCFC-123b).

### Item 6.

The method according to any one of Items 1 to 5, wherein a hydrogenation reaction is performed in step A.

### Item 7.

The method according to Item 6, wherein the hydrogenation reaction is performed at a temperature of 150°C or higher and 350°C or lower.

### Item 8.

The method according to Item 6 or 7, wherein the hydrogenation reaction is performed by adding hydrogen to the chlorofluoroethane at an H₂/chlorofluoroethane molar ratio of 1 or more and 20 or less.

### Item 9.

The method according to any one of Items 6 to 8, wherein the hydrogenation reaction is performed in a gas phase.

### Item 10.

A method for producing a fluoroolefin, further comprising step B of subjecting a fluoroethane obtained by the production method of any one of Items 1 to 9 to a dehydrofluorination reaction to obtain a fluoroolefin.

### Advantageous Effects of Invention

The method for producing a fluoroethane according to the present disclosure described above enables a fluoroethane to be obtained in a high yield.

### Description of Embodiments

In the present specification, the terms "comprise," "contain," and "include" encompass the concepts of comprising, consisting essentially of, and consisting of.

### 1. Method for Producing Fluoroethane

The method for producing a fluoroethane of the present disclosure is a method for producing a fluoroethane represented by the following formula (1)

CH₂X¹CHX²X³ (1)

wherein X¹, X², and X³ each independently represent a hydrogen atom or a fluorine atom, and at least one of X¹, X², and X³ represents a fluorine atom.

The method for producing a fluoroethane of the present disclosure comprises step A of obtaining the fluoroethane from a chlorofluoroethane represented by the following formula (2) in the presence of a catalyst:

CX⁴ClFCX⁵X⁶X⁷ (2)

wherein X⁴, X⁵, X⁶, and X⁷ each independently represent a hydrogen atom, a fluorine atom, or a chlorine atom, and at least one of X⁵, X⁶, and X⁷ represents a hydrogen atom.

Step A is performed as a hydrogenation reaction, and is preferably performed in the presence of a catalyst in a reactor. A common raw material is used as a hydrogenation agent. In addition to hydrogen gas, hydrogenation agents, such as hydrazine, can be used, and hydrogen gas is preferably used.

As the catalyst, it is preferable to use a metal catalyst, more preferable to use Ni, Pd, Pt, Rh, or Ru, and particularly preferable to use Pd.

Furthermore, the catalyst is more preferably supported on a carrier for use. The carrier is not limited, and a wide range of known carriers can be used. Examples of constituent materials of the carrier include carbon-based materials, such as activated carbon, amorphous carbon, graphite, and diamond, porous aluminosilicate typified by zeolite, aluminophosphate, aluminum oxide, silicon oxide, titanium oxide, zirconium oxide, zinc oxide, aluminum fluoride, and the like. These may be used singly or in a combination of two or more. Among these, it is preferable to use a carbon-based carrier by employing a carbon-based material described above.

The amount of metal supported in the catalyst is preferably 0.2 mass% or more, more preferably 0.5 mass% or more, and even more preferably 1.0 mass% or more, based on the total mass of the carrier and the metal taken as 100 mass%. Further, the mass of the catalyst is preferably 5 mass% or less, and more preferably 4 mass% or less, based on the total mass of the carrier and the catalyst taken as 100 mass%.

The method for preparing the catalyst is not limited, and a wide range of known preparation methods can be used. For example, a catalyst comprising a noble metal supported on a carrier can be obtained by a method comprising immersing a carrier in a solution containing a noble metal to impregnate the carrier with the solution, and then, if necessary, performing neutralization, drying, calcination, etc. In this case, the amount of noble metal supported on the carrier can be controlled by adjusting the concentration of the solution, the impregnation time, etc.

The fluoroethane represented by formula (1) is preferably 1,1,2-trifluoroethane (HFC-143), 1,1-difluoroethane (HFC-152a), 1,2-difluoroethane (HFC-152), or fluoroethane (HFC-161).

According to the production method of the present disclosure, it is possible to produce only one type of these fluoroethanes or to produce multiple types of these fluoroethanes simultaneously. When multiple types of fluoroethanes are produced simultaneously, it is also preferable to separate them into individual fluoroethanes using a rectification column or the like.

The chlorofluoroethane represented by formula (2) is not limited, and examples include 1,2-dichloro-1,1,2-trifluoroethane (HCFC-123a), 1,1-dichloro-1,2,2-trifluoroethane (HCFC-123b), and 1,1,2-trichloro-1,2,2-trifluoroethane (CFC-113). These may be used singly or in a combination of two or more. For example, the use of only one of HCFC-123a or HCFC-123b alone, or the use of a mixture of HCFC-123a and HCFC-123b is also a preferred embodiment.

The reaction performed in step A is not limited as long as it is a reaction capable of replacing fluorine atom(s) or chlorine atom(s) attached to carbon atom(s) in a fluoroethane by hydrogen atom(s). Specifically, it is preferable to perform a hydrogenation reaction.

When a hydrogenation reaction is performed in step A, hydrogen is added to the chlorofluoroethane represented by formula (2) at an H₂/chlorofluoroethane molar ratio of preferably 1 or more, more preferably 2 or more, even more preferably 3 or more, and particularly preferably 5 or more. The molar ratio is also preferably 20 or less, more preferably 18 or less, even more preferably 15 or less, and particularly preferably 13 or less. By setting the H₂/chlorofluoroethane ratio to be equal to or greater than the lower limit, the decrease in catalyst activity can be suppressed, and the selectivity of the desired product can be improved. Meanwhile, by setting the H₂/chlorofluoroethane ratio to be equal to or less than the upper limit, excessive hydrogen generation can be suppressed, and the reaction efficiency and yield can be improved.

In this case, the temperature condition for allowing the hydrogenation reaction to proceed is preferably 150°C or higher, and more preferably 180°C or higher. The temperature condition is also preferably 350°C or lower, and more preferably 320°C or lower. By setting the temperature condition to be equal to or greater than the lower limit, suitable conversion of the starting material and suitable selectivity of the desired product can be obtained. Meanwhile, by setting the temperature condition to be equal to or less than the upper limit, the progress of side reactions can be suppressed, the deterioration of the catalyst can be suppressed, and the yield can be improved.

The contact time represented by W/FO, i.e., the ratio of the catalyst amount (mass) in the reaction system when a hydrogenation reaction is performed W (g) to the total flow rate of the chlorofluoroethane represented by formula (2) and hydrogen gas introduced into the reaction system F0 (cc/sec), is preferably 0.1 or more (g·sec/cc), more preferably 0.5 or more (g·sec/cc), and even more preferably 1.0 or more (g·sec/cc). The contact time is also preferably 20 or less (g·sec/cc), more preferably 15 or less (g·sec/cc), and even more preferably 10 or less (g·sec/cc). By setting the contact time within the above numerical range, high conversion and high selectivity can be obtained, thereby making the process efficient.

In the reaction of step A, the reaction system may contain a compound other than the chlorofluoroethane represented by formula (2). For example, the reaction system may contain chlorotrifluoroethylene (CTFE) and/or 1,1,2-trifluoroethylene (HFO-1123).

The reaction in step A proceeds, for example, according to the following reaction scheme 1.

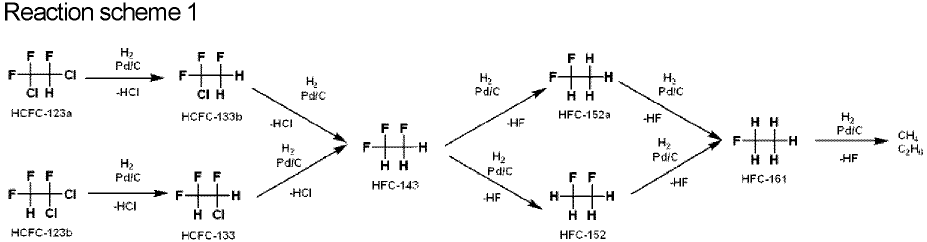

In the above reaction scheme, both HCFC-133 and HCFC-133b can be converted to HFC-143, indicating that the formation of any of these useful substances that can be converted to the desired product can also contribute to an improvement in selectivity.

### 2. Method for Producing Fluoroolefin

The method for producing a fluoroolefin of the present disclosure comprises a step of obtaining a fluoroolefin by a dehydrofluorination reaction of a fluoroethane obtained by the method for producing a fluoroethane described above. Hereinafter, this step is defined as step B in the present specification.

In the method for producing a fluoroolefin of the present disclosure, preferably, a fluoroolefin represented by the following formula (3):

CX⁸X⁹=CX¹⁰X¹¹ (3)

wherein X⁸, X⁹, X¹⁰, and X¹¹ are the same or different, and each represents a hydrogen atom, a fluorine atom, or a chlorine atom;
at least one of X⁸, X⁹, X¹⁰, and X¹¹ represents a hydrogen atom;
and at least one of X⁸, X⁹, X¹⁰, and X¹¹ represents a fluorine atom, can be obtained by subjecting a fluoroethane represented by the following formula (1):

   CH₂X¹CHX²X³ (1)
wherein X¹, X², and X³ each independently represents a hydrogen atom or a fluorine atom, and at least one of X¹, X², and X³ represents a fluorine atom, to a dehydrofluorination reaction.

In step B, the method for the dehydrofluorination reaction is not limited. For example, the dehydrofluorination reaction may be performed under conditions that are the same as or similar to those of known dehydrofluorination reactions. For example, the dehydrofluorination reaction may be performed in a gas phase, in the presence of a catalyst for dehydrofluorination.

In the method for producing a fluoroolefin of the present disclosure, the dehydrofluorination reaction when 1,1,2-trifluoroethane (HFC-143) is used as a fluoroethane is performed according to the following reaction scheme.

CF₂HCFH₂ → CHF=CHF + HF

The catalyst for dehydrofluorination is not limited, and a wide range of known catalysts can be used. Examples include chromium oxide, fluorinated chromium oxide, aluminum oxide, fluorinated aluminum oxide, and the like.

The catalyst for dehydrofluorination is preferably supported on a carrier. Examples of carriers include carbon, alumina (Al₂O₃), zirconia (ZrO₂), silica (SiO₂), titania (TiO₂), and the like. As carbon, activated carbon, amorphous carbon, graphite, diamond, or the like can be used.

The dehydrofluorination reaction in step B may also be performed in the presence of an oxidizing agent. Examples of oxidizing agents include oxygen, chlorine, bromine, iodine, and the like. Oxygen is particularly preferable. The concentration of the oxidizing agent is not limited; and may be, for example, the same as or similar to that in known dehydrofluorination reactions.

The reaction temperature in the dehydrofluorination reaction is also not limited; and may be the same as or similar to that in known dehydrofluorination reactions. For example, the reaction temperature in the dehydrofluorination reaction may be 300°C or higher, preferably 320°C or higher, more preferably 340°C or higher, and particularly preferably 350°C or higher. The reaction temperature in the dehydrofluorination reaction may also be 600°C or lower, preferably 550°C or lower, more preferably 500°C or lower, and particularly preferably 450°C or lower.

The reaction time of the dehydrofluorination reaction and the pressure during the reaction are also not limited, and a wide range of known conditions can be adopted. The dehydrofluorination reaction may also be performed either in the presence of an inert gas, or in the presence of air. As inert gases, in addition to helium, nitrogen, and argon, fluorocarbons and hydrofluorocarbons such as HFC-23, FC-14, FC-116, HFC-152a, and HFC-161, which are fluorine compounds that are not involved in the reaction, can be used. The dehydrofluorination reaction may be performed either continuously or batch-wise.

The method for producing a fluoroolefin of the present disclosure may also comprise, if necessary, other steps in addition to step B. Also in the method for producing a fluoroolefin of the present disclosure, the starting material can be separated from the crude product obtained in the production method and recycled.

The desired fluoroolefin, for example, a compound represented by formula (3), is obtained by step B. In the dehydrofluorination step, one or more fluoroolefins are obtained.

The resulting fluoroolefin can depend on the fluoroethane used in the dehydrofluorination step. Examples of fluoroolefins include 1,2-difluoroethylene (HFO-1132), 1,1-difluoroethylene (HFO-1132a), trifluoroethylene (HFO-1123), and the like.

In the method for producing a fluoroolefin of the present disclosure, when HFC-143 is used as a fluoroethane, the resulting fluoroolefin is HFO-1132. In Production Method according to the present disclosure, when HFC-143a is used as a fluoroethane, the resulting fluoroolefin is HFO-1132a. In the method for producing a fluoroolefin of the present disclosure, when HFC-134 is used as a fluoroethane, the resulting fluoroolefin is HFO-1123. HFO-1132 can include trans-1,2-difluoroethylene [(E)-HFO-1132] and cis-1,2-difluoroethylene [(Z)-HFO-1132].

To obtain a fluoroolefin by the method for producing a fluoroolefin of the present disclosure described above, the method for producing a fluoroethane of the present disclosure described above and the method for producing a fluoroolefin of the present disclosure may be performed consecutively or may be performed independently.

Embodiments of the present disclosure are described above; however, the present disclosure is not limited to these examples. Of course, the present disclosure can be embodied in various forms without departing from the gist of the present disclosure.

### Examples

Embodiments of the present disclosure are described in more detail below based on Examples; however, the present disclosure is not limited thereto.

### Example 1

0.2 kg of an activated carbon catalyst in which 2 mass% of Pd was supported was placed in a Hastelloy C reaction tube with an inner diameter of 50 mm and dried at 250°C for 3 hours through nitrogen purge. Thereafter, the temperature of the reactor was set to 200°C, HCFC-123a at 2.7 g/min and hydrogen at 3600 Nml/min were supplied, and a reduction reaction was performed. At this time, W/FO was 3, and the H₂/HCFC-123a molar ratio was 9. The outlet gas was washed with water, dried over calcium chloride, and then analyzed by GC (GC-2030, produced by Shimadzu Corporation). The results showed that HFC-143 was obtained in a high yield (conversion of HCFC-123a: 28.8%, selectivity of HFC-143 and useful substances that can be converted to HFC-143: 95.6%).

### Example 2

0.2 kg of an activated carbon catalyst in which 2 mass% of Pd was supported was placed in a Hastelloy C reaction tube with an inner diameter of 50 mm and dried at 250°C for 3 hours through nitrogen purge. Thereafter, the temperature of the reactor was set to 250°C, HCFC-123a at 2.7 g/min and hydrogen at 3600 Nml/min were supplied, and a reduction reaction was performed. At this time, W/FO was 3, and the H₂/HCFC-123a molar ratio was 9. The outlet gas was washed with water, dried over calcium chloride, and then analyzed by GC (GC-2030, produced by Shimadzu Corporation). The results showed that HFC-143 was obtained in a high yield (conversion of HCFC-123a: 70.4%, selectivity of HFC-143 and useful substances that can be converted to HFC-143: 97.6%).

### Example 3

0.2 kg of an activated carbon catalyst in which 2 mass% of Pd was supported was placed in a Hastelloy C reaction tube with an inner diameter of 50 mm and dried at 250°C for 3 hours through nitrogen purge. Thereafter, the temperature of the reactor was set to 300°C, HCFC-123a at 2.7 g/min and hydrogen at 3600 Nml/min were supplied, and a reduction reaction was performed. At this time, W/FO was 3, and the H₂/HCFC-123a molar ratio was 9. The outlet gas was washed with water, dried over calcium chloride, and then analyzed by GC (GC-2030 produced by Shimadzu Corporation). The results showed that HFC-143 was obtained in a high yield (conversion of HCFC-123a: 98.7%, selectivity of HFC-143 and useful substances that can be converted to HFC-143: 98.3%).

The results of Examples 1 to 3 above are summarized in Table 1 below.

**Table 1**

| | Example 1 | Example 2 | Example 3 |
|---|---|---|---|
| Reaction temperature (°C) | 200 | 250 | 300 |
| Conversion of 123a | 28.8 | 70.4 | 98.7 |
| Selectivity of 143 | 14.0 | 17.6 | 47.2 |
| Selectivity of 133b | 80.0 | 59.0 | 39.3 |
| Selectivity of 133 | 1.6 | 21.0 | 11.8 |
| Selectivity of HFC-143 and useful substances that can be converted to HFC-143 | 95.6 | 97.6 | 98.3 |

## Claims

1. A method for producing a fluoroethane represented by the following formula (1):
CH₂X¹CHX²X³ (1)
wherein X¹, X², and X³ each independently represent a hydrogen atom or a fluorine atom, and at least one of X¹, X², and X³ represents a fluorine atom,
the method comprising step A of obtaining the fluoroethane from a chlorofluoroethane represented by the following formula (2) in the presence of a catalyst:
CX⁴ClFCX⁵X⁶X⁷ (2)
wherein X⁴, X⁵, X⁶, and X⁷ each independently represent a hydrogen atom, a fluorine atom, or a chlorine atom, and at least one of X⁵, X⁶, and X⁷ represents a hydrogen atom.

2. The method according to claim 1, wherein the catalyst is at least one member selected from the group consisting of Ni, Pd, Pt, Rh, and Ru.

3. The method according to claim 1 or 2, wherein the catalyst is supported on a carrier, and the carrier is a carbon-based carrier.

4. The method according to claim 1 or 2, wherein the fluoroethane is 1,1,2-trifluoroethane (HFC-143), 1,1-difluoroethane (HFC-152a), 1,2-difluoroethane (HFC-152), or fluoroethane (HFC-161).

5. The method according to claim 1 or 2, wherein the chlorofluoroethane is 1,2-dichloro-1,1,2-trifluoroethane (HCFC-123a) or 1,1-dichloro-1,2,2-trifluoroethane (HCFC-123b).

6. The method according to claim 1 or 2, wherein a hydrogenation reaction is performed in step A.

7. The method according to claim 6, wherein the hydrogenation reaction is performed at a temperature of 150°C or higher and 350°C or lower.

8. The method according to claim 6, wherein the hydrogenation reaction is performed by adding hydrogen to the chlorofluoroethane at an H₂/chlorofluoroethane molar ratio of 1 or more and 20 or less.

9. The method according to claim 6, wherein the hydrogenation reaction is performed in a gas phase.

10. A method for producing a fluoroolefin, further comprising step B of subjecting a fluoroethane obtained by the production method of claim 1 or 2 to a dehydrofluorination reaction to obtain a fluoroolefin.
